# EUROPÄISCHE PATENTANMELDUNG

(11) **EP 2 389 940 A2**
(43) Veröffentlichungstag der Anmeldung: **30.11.2011**
(21) Anmeldenummer: 11003069.9
(22) Anmeldetag: 12.04.2011
(51) Int. Cl.: A61K 31/728, A61M 31/00

(54) **Vaginalapplikator mit zwei Auslassöffnungen**

(30) Priorität: 16.04.2010 DE 102010015350
(71) Anmelder: Zimmermann, Michael, 65931 Frankfurt am Main (DE)
(72) Erfinder: Zimmermann, Michael, 65931 Frankfurt am Main (DE)
(74) Vertreter: Metten, Karl-Heinz

(57) **Zusammenfassung**

Die vorliegende Erfindung betrifft einen Vaginalapplikator, umfassend mindestens eine erste Aufbewahrungseinheit, enthaltend mindestens ein Mucopolysaccharid, insbesondere Hyaluronsäure und/oder ein Derivat und/oder Salz der Hyaluronsäure, mit einer ersten Auslassöffnung und mindestens eine zweite Aufbewahrungseinheit, enthaltend mindestens eine Säure, insbesondere organische Säure, mit einer zweiten Auslassöffnung, wobei die erste und die zweite Auslassöffnung benachbart angeordnet sind unter Ausbildung einer ersten Ausgabeeinheit zur gemeinsamen Applikation von Mucopolysaccharid und Säure in die Vagina.

## Beschreibung

Die vorliegende Erfindung betrifft Vaginalapplikatoren zur Applizierung von mindestens einem Mucopolysaccharid und mindestens einer Säure.

Bei der gesunden Frau wird die Vagina durch ein Transsudat aus dem Vaginalepithel sowie durch ein Sekret aus den Bartholinschen Drüsen befeuchtet. Ein Mangel an Östrogen in den Epithelzellen der Scheide führt in der Regel zum Untergang des Gewebes mit der Folge, dass die Transsudation versiegt. Vaginale Trockenheit verursacht Irritation, Juckreiz, Epithelschädigungen, Schmerzen, Entzündungen und ein gestörtes Sexualempfmden. Änderungen bei der Östrogenkonzentration beobachtet man als Folge der Wechseljahre, bei bzw. nach einer Schwangerschaft, bei Medikamenten- bzw. Hormoneinnahme, nach der Ovarektomie sowie nach der Krebsbehandlung von Mammakarzinompatientinnen. Nicht selten verbietet sich eine Östrogenzugabe, um die genannten Symptome abzustellen, beispielsweise bei Mammakarzinompatientinnen, so dass man auf alternative Behandlungskonzepte, soweit verfügbar, angewiesen ist.

Der Mangel an Östrogen im Vaginalepithel, führt zum Versiegen des Glycogens und damit regelmäßig auch zu einem erhöhten pH-Wert im Transsudat. Denn die in der Vagina vorhandenen Lactobazillen setzen die in den Vaginalzellen aus Glycogen gebildete Glucose zu Milchsäure und anderen organischen Säuren um. In der Regel wird auf diese Weise ein physiologischer pH-Wert von 3,8 bis 4,5 im Transsudat eingestellt. Gleichzeitig ist ein Teil der Lactobazillen in der Lage, H₂O₂ (Wasserstoffperoxid) zu bilden. Die mit der Abnahme der Östrogenmenge einhergehende erniedrigte Milchsäureproduktion in der Scheidenwand führt zu einer Vaginalflora, die nicht mehr ausreichend vor Infektionen schützt. Die Anfälligkeit gegenüber bakteriellen Vaginosen nimmt zu (Mendling, Vaginose, Vaginitis, Zervizitis und Salpingitis, Springer, 2. Auflage, 2006).

Da der Östrogenspiegel zyklischen Schwankungen unterliegt und sich in verschiedenen Lebenssituationen auch längerfristig verändern kann und man nicht in jedem Fall auf eine Hormonbehandlung angewiesen sein möchte, hat es nicht an Versuchen gefehlt, das Problem der verminderten Transsudation und des Anstiegs des pH-Werts auf andere Art und Weise in den Griff zu bekommen.

Sehr viele Vaginalpräparate sind dafür vorgesehen, entweder das Problem der Trockenheit der Scheide oder das Problem des mangelnden Säureschutzes zu linderen. Hierbei wird zur Linderung der Trockenheit u.a. auf Präparate enthaltend Hyaluronsäure zurückgegriffen. Die Hyaluronsäure ist ein saures Mucopolysaccharid, das zu den wichtigsten Strukturelementen der menschlichen Haut gehört. Es stellt ein Polysaccharid dar, das aus sich wiederholenden, 1,3-glycosidisch verknüpften Glucuronsäure- und N-Acetylglucosamin-Einheiten aufgebaut ist. Hyaluronsäure ist stark hygroskopisch und kann bis zu 3 Liter Wasser/g binden. Hyaluronsäure beeinflusst den Aufbau der extracellulären Matrix und stimuliert die Fibroblastenproliferation. Hyaluronsäure ist in jeder Phase der Gewebereparatur der Haut beteiligt. Der fördernde Einfluss der Hyaluronsäure bei der Hydratisierung von Gewebe wie auch bei der Gewebereparatur ist bekannt und z.B. bei G. Morali et al. beschrieben (Drug Res. 2006, 56, 3, 230-238).

Vaginalpräparate auf Milchsäurebasis zwecks Verminderung des Säuregrades der Vaginalflüssigkeit zur Verbesserung der Infektabwehr einzusetzen, ist ebenfalls bekannt. Der natürliche pH-Wert der Vaginalflüssigkeit liegt im Bereich von 3,8 bis 4,5. Ein derart saures Milieu wird von fakultativ pathogenen Keimen regelmäßig nicht vertragen. Der Einsatz eines Milchsäuregels für die Infektprävention hat sich zwar als zweckmäßig erwiesen (Andersch et al., Gynecol obstet invest. 1986), eine dauerhafte Hydratisierung ist damit aber nicht verbunden.

Eine pharmazeutische Formulierung zur Verabreichung durch eine Schleimhautmembran erhält man gemäß der EP 1 053 011 B1 durch Zugabe einer mizellären Arzneimittelformulierung zu mindestens einer absorptionsverstärkenden Verbindung ausgewählt aus der Gruppe bestehend aus Lecithin, Hyaluronsäure, Octylphenoxypolyethoxyethanol, Glycolsäure, Milchsäure, Kamilleextrakt, Gurkenextrakt, Ölsäure, Linolensäure, Borretschöl, Nachtrosenöl, Menthol, Trihydroxyoxocholanylglycin, Glycerin, Polyglycerin, Lysin, Polydocanolalkylethern und Triolein. Aus der WO 96/29867 A2 geht eine Formulierung zur topischen Anwendung auf Schleimhäuten hervor, welche antiseptisch wirksam ist. Eine wässrige antiseptische Formulierung, enthaltend einen antimikrobiell wirksamen Alkohol oder Glycol sowie eine die antimikrobielle Wirkung verlängernde stickstoffhaltige Verbindung, kann hierbei ebenfalls mit Hyaluronsäure ausgestattet sein. Gemäß der WO 2005/063161 A1 kann Hyaluronsäure alternativ zu Glycerin, Silikonwachs, pflanzlichen Wachsen oder Paraffinwachs eingesetzt werden, um die Gleitfähigkeit von Tampons zu verbessern.

Aus der WO 2006/089561 A1 ist bekannt, organische Säuren in Vaginalpräparaten einzusetzen, um den pH-Wert in der Vagina zu verringern. Geeignete organische Säuren können dabei z.B. ausgewählt werden aus Essigsäure, Glycolsäure, Propionsäure, Milchsäure, Buttersäure, Fumarsäure, Äpfelsäure, Benzoesäure, Hydroxybenzoesäure, Para-Aminobenzoesäure, Zitronensäure, Mandelsäure, Malonsäure, Oxalsäure, Salicylsäure, Adipinsäure, Weinsäure, Ascorbinsäure, Retinolsäure, Glucuronsäure, Iduronsäure, Hyaluronsäure, Nikotinsäure, Aminosäuren wie Valin und Arginin, und Fettsäuren wie Ölsäure, Linolsäure, Linolensäure und Arachidonsäure.

Zur Behandlung einer trockenen Scheide wird in der WO 2007/085327 A1 die Verwendung von Lupulus, vorzugsweise in Form eines Extraktes vorgeschlagen. Die Lupulus enthaltende Formulierung kann z.B. in Form einer Creme, eines Gels, eines Schaums, einer Emulsion, einer Lösung oder einer Suspension vorliegen. Als weitere Verbindung kann alternativ zu mit Polyalkenylpolyethern quervernetzter Polyacrylsäure, einem Polyoxyethylen/Polyoxypropylen-Blockpolymeren, Cellulose, Alginaten und Collagen auch Hyaluronsäure zugegen sein. Als Hydratisierungsagentien werden Alkohole, polyhydroxylierte Gylcole, Mucopolysaccharide, Milchsäure, 2-Pyrrolidon-Carbonsäuren sowie pflanzliche Öle und Glycole genannt.

Die DE 692 06 913 T2 befasst sich mit einer Liposomenformulierung für eine hydratisierende und/oder die Gleitfähigkeit erhöhende Vaginalbehandlung. Diese Formulierung hat aus einer oder mehreren Phospholipid-, Sphingolipid-, Glycolipid- oder Protheolipidsubstanzen, bioadhäsiven Polymeren und einer kontinuierlichen wässrigen Lösungsmittelphase zu bestehen. Als bioadhäsive Polymere werden Carbopol, Cellulose- und Acrylsäurederivate, Alginate, Collagen und Hyaluronsäure genannt.

Aus der DE 691 20 933 T2 geht die Verwendung von Glycosaminoglycanen zur Herstellung einer topischen pharmazeutischen Zusammensetzung zur Prävention und Therapie von Erkrankungen im zervikalen Vaginalbereich hervor. Geeignete Glycosaminoglycane können ausgewählt werden aus Heparansulfat, Dermatansulfat, Chondroitinsulfat A und C und Hyaluronsäure. Die topische Zusammensetzung kann in Form von Ovuli, Cremes, Pomaden, Lotionen und Salben vorliegen.

Die DE 32 00 766 A1 beschreibt ein Präparat, mit dem die Absorption eines Arzneimittels durch ein Organ des Gastroinstestinaltrakts, wie Colon- oder Rectum, oder durch die Vagina gelingen soll. Dieses Präparat hat neben einem Wirkstoff eine wasserlösliche Verbindung mit chelatbildender Aktivität zu enthalten. Hierbei kann es sich um wasserlösliche Polysaccharidverbindungen, wasserlösliche Cellulosederivate, wasserlösliche Stärkederivate, Dextranderivate und wasserlösliche Polypeptidverbindungen handeln. Alternativ kann auch eine niedermolekulare wasserlösliche Verbindung mit einem oder mehreren chelatbildenden Liganden eingesetzt werden. Als solche niedermolekulare wasserlösliche Verbindungen mit einem chelatbildenden Liganden werden u.a. genannt Bernsteinsäure, Adipinsäure, Brenztraubensäure, Acetoessigsäure, Buttersäure, Salicylsäure, Zitronensäure, Äpfelsäure oder DL-Asparaginsäure.

Vaginalsuppositorien, enthaltend ein Peptid mit LH-RH-Wirkung in einem Gemisch mit einem öligen Träger werden in der DE 28 36 631 A1 beschrieben. Indem man eine wasserlösliche aliphatische Carbonsäure mit 2 bis 6 C-Atomen einarbeitet, soll eine gesteigerte Resorption des Wirkstoffs erzielt werden. Als geeignete wasserlösliche aliphatische Carbonsäuren werden Essigsäure, Milchsäure, Ascorbinsäure, Bernsteinsäure, Weinsäure, Malonsäure, Buttersäure, Äpfelsäure, Asparaginsäure und Zitronensäure ausdrücklich genannt.

Da die Symptome der trockenen Scheide und eines erhöhten pH-Wertes der Vaginalflüssigkeit meistens miteinander einhergehen, hat es nicht an Versuchen gefehlt, Vaginaltherapeutika zur vorbeugenden Behandlung von bakteriellen Vaginalinfektionen zur Verfügung zu stellen, die auf einer Kombination von z.B. hyaluronsäurehaltigen Produkten und Milchsäure basieren. Eine Kombination dieser beiden Wirkstoffe scheitert daran, dass die Hyaluronsäure unter sauren Bedingungen hydrolysiert wird. Verwendet man Vaginaltherapeutika auf Hyaluronsäurebasis mit neutralem pH-Wert, wird dadurch das Infektionsrisiko noch gesteigert, da die in der Vaginalflüssigkeit vorhandene Restsäure dann zumeist vollständig neutralisiert wird.

Gegenwärtig befindet sich als Kombinationspräparat enthaltend Hyaluronsäure und Milchsäure ein Vaginalzäpfchen mit dem Handelsnamen Premeno Duo^{®} auf dem Markt. Bei diesem Produkt liegen Hyaluronsäure und Milchsäure nebeneinander in einer schmelzbaren fetthaltigen Substanz vor. Das "Tragen" einer solchen flüssigen Fettphase wird mitunter als unangenehm empfunden.

Es wäre somit wünschenswert, auf solche Applikationsformen für eine kombinierte Verwendung von z.B. Hyaluronsäure und Milchsäure zurückgreifen zu können, die nicht auf einer Fettzusammensetzung basieren.

Demgemäß lag der vorliegenden Erfindung die Aufgabe zu Grunde, ein Vaginaltherapeutikum zur Verfügung zu stellen, das nicht mit den Nachteilen des Stands der Technik behaftet ist und das insbesondere die kombinierte Gabe von z.B. Hyaluronsäure und Milchsäure erlaubt, ohne während der Phasen der Herstellung und/oder Lagerung die Gefahr des säurebedingten hydrolytischen Abbaus der Hyaluronsäure in Kauf nehmen und ohne Zugeständnisse beim Applikationskomfort machen zu müssen.

Demgemäß wurde ein Vaginalapplikator gefunden, umfassend mindestens eine erste Aufbewahrungseinheit, enthaltend mindestens ein Mucopolysaccharid, insbesondere Hyaluronsäure und/oder ein Derivat und/oder Salz der Hyaluronsäure, mit einer ersten Auslassöffnung und mindestens eine zweite Aufbewahrungseinheit, enthaltend mindestens eine Säure, insbesondere organische Säure, mit einer zweiten Auslassöffnung, wobei die erste und die zweite Auslassöffnung benachbart angeordnet sind unter Ausbildung einer ersten Ausgabeeinheit zur gemeinsamen Applikation von Mucopolysaccharid und Säure in die Vagina, auch Vaginalapplikator bzw. vaginale Applikationsform gemäß einer ersten Ausgestaltung genannt.

In einer zweckmäßigen Ausführungsform sind die erste und/oder die zweite Aufbewahrungseinheit zumindest bereichsweise flexibel gestaltet. D.h. die Wandungen von erster und zweiter Aufbewahrungseinheit sind derart nachgiebig, dass durch Anwendung von Druck die erste bzw. die zweite Aufbewahrungseinheit verformt werden können, wodurch eine Freigabe bzw. ein Herauspressen von Mucopolysaccharid und Säure aus der ersten bzw. zweiten Aufbewahrungseinheit gelingt. Bei der ersten Ausgestaltung eines erfindungsgemäßen Vaginalapplikators sind die erste und zweite Aufbewahrungseinheit bzw. die diese Aufbewahrungseinheiten bildenden Wandungen stabil in Gegenwart von Mucopolysaccharid bzw. Säure. Als Materialien für die erste und zweite Aufbewahrungseinheit kommen beispielsweise Polymere, insbesondere thermoplastische Polymere, sowie Mischungen solcher Polymere untereinander und/oder mit weiteren anorganischen und organischen Materialien in Frage. In einer möglichen Ausführungsform liegen dabei die erste und die zweite Aufbewahrungseinheit nebeneinander vor und können hierbei über eine gemeinsame Zwischen- bzw. Trennwand verfügen. Die erste und die zweite Auslassöffnung sind vorzugsweise auf gleicher Höhe angebracht, d.h. die Öffnungsquerschnitte von erster und zweiter Auslassöffnung liegen vorzugsweise in einer Ebene.

Die erste Ausgabeeinheit ist in Form und Größe zweckmäßigerweise derart gewählt, dass eine unproblematische gleichzeitige Applikation von Mucopolysaccharid und Säure gelingt. Demgemäß kann in einer weiteren, bevorzugten Ausgestaltung vorgesehen sei, dass die erste und zweite Auslassöffnung nebeneinander vorliegen, insbesondere eine gemeinsame Trennwand aufweisen.

Besonders vorteilhafte Resultate stellen sich auch dadurch ein, dass die erste Aufbewahrungseinheit zumindest bereichsweise innerhalb der zweiten Aufbewahrungseinheit angeordnet ist und/oder dass die erste Auslassöffnung zumindest bereichsweise im Umfang der zweiten Auslassöffnung vorliegt oder dass die zweite Aufbewahrungseinheit zumindest bereichsweise innerhalb der ersten Aufbewahrungseinheit vorliegt und/oder dass die zweite Auslassöffnung zumindest bereichsweise im Umfang der ersten Auslassöffnung vorliegt.

Liegt die erste Aufbewahrungseinheit beispielsweise in der zweiten Aufbewahrungseinheit vor, hat es sich als vorteilhaft erwiesen, wenn der Boden der ersten Aufbewahrungseinheit fest mit dem Boden der zweiten Aufbewahrungseinheit verbunden ist. Dieses kann auch über einen Abstandshalter geschehen. Selbstverständlich ist es hierbei auch möglich, dass die erste und die zweite Aufbewahrungseinheit über einen einheitlichen Boden verfügen, d.h. die Wandungen der ersten Aufbewahrungseinheit gehen flüssigkeitsdicht in den Boden der zweiten Aufbewahrungseinheit über. Auch bei der vorangehenden geschilderten Ausführungsform ist es vorteilhaft, wenn die durch die ersten und zweiten Auslassöffnungen aufgespannten Flächen in einer gemeinsamen Ebene liegen. Dieses ist jedoch nicht zwingend erforderlich. So kann die Auslassöffnung der ersten Aufbewahrungseinheit relativ ober- oder unterhalb der durch die zweite Auslassöffnung aufgespannten Ebene liegen.

Die erste Ausgestaltung eines erfindungsgemäßen Vaginalapplikators kann ferner eine erste Applikationshilfe, die mit der ersten Ausgabeeinheit verbunden oder verbindbar ist, umfassen. Bei dieser ersten Applikationshilfe kann es sich z.B. um einen auf die erste Ausgabeeinheit aufstülpbaren oder aufschraubbaren röhrenförmigen Fortsatz handeln, mit dessen Hilfe die gleichzeitige Applizierung von Mucopolysaccharid und Säure über den gesamten in Frage kommenden Vaginalbereich besonders gut gelingt.

In einer weiteren Ausführungsform können zwecks verbesserter Vermischung von Mucopolysaccharid und Säure die erste Ausgabeeinheit und/oder die erste Applikationshilfe mindestens ein Mischelement enthalten. Hierbei können aus der Strömungstechnik bekannte Mischelemente zum Einsatz kommen, beispielsweise statische Mischer.

Die der Erfindung zu Grunde liegende Aufgabe wird des weiteren gelöst durch einen Vaginalapplikator in Form eines Applikationskits für die gleichzeitige Applikation von Mucopolysaccharid und Säure, auch Vaginalapplikator bzw. vaginale Applikationsform gemäß einer zweiten Ausgestaltung genannt, umfassend ein kombiniertes Aufbewahrungsbehältnis, enthaltend eine erste Aufbewahrungseinheit, enthaltend mindestens ein Mucopolysaccharid, insbesondere Hyaluronsäure und/oder ein Derivat und/oder Salz der Hyaluronsäure, mit einer ersten Auslassöffnung und mindestens eine zweite Aufbewahrungseinheit, enthaltend mindestens eine Säure, insbesondere organische Säure, mit einer zweiten Auslassöffnung, wobei diese erste und zweite Auslassöffhungen benachbart angeordnet sind unter Ausbildung einer zweiten Ausgabeeinheit, sowie mindestens eine zweite Applikationshilfe, umfassend einen Öffnungsaufsatz, der reversibel dichtend mit der zweiten Ausgabeeinheit verbunden oder verbindbar ist, und über mindestens eine Applikatorvorrichtung verfügt zur gemeinsamen Applikation von Mucopolysaccharid und Säure in die Vagina über den Öffnungsaufsatz.

Das kombinierte Aufbewahrungsbehältnis des erfindungsgemäßen Vaginalapplikators gemäß der zweiten Ausgestaltung unterscheidet sich von dem erfindungsgemäßen Vaginalapplikator gemäß der ersten Ausgestaltung im wesentlich dadurch, dass die zweite Ausgabeeinheit des Aufbewahrungsbehältnisses als solche, beispielsweise auf Grund ihrer Form, Größe und/oder Länge, nicht geeignet ist zur gemeinsamen Applikation von Mucopolysaccharid und Säure in die Vagina. Vielmehr bedarf es hierfür der zweiten Applikationshilfe, welche reversibel dichtend mit der zweiten Ausgabeeinheit verbunden oder verbindbar ist. Jedenfalls diese zweite Applikationseinheit verfügt über eine dritte Ausgabeeinheit, mit deren Hilfe die gemeinsame Applikation von Mucopolysaccharid und Säure in die Vagina gelingt.

Die der Erfindung zu Grunde liegende Aufgabe wird des weiteren gelöst durch einen Vaginalapplikator, auch Vaginalapplikator bzw. vaginale Applikationsform gemäß einer dritten Ausgestaltung genannt, enthaltend mindestens eine erste Aufbewahrungs- und Freisetzungseinheit, enthaltend mindestens ein Mucopolysaccharid, insbesondere Hyaluronsäure und/oder ein Derivat und/oder Salz der Hyaluronsäure, mit einer ersten Wandung und mindestens eine zweite Aufbewahrungs- und Freisetzungseinheit, enthaltend mindestens eine Säure, insbesondere organische Säure, mit einer zweiten Wandung, wobei Mucopolysaccharid und Säure durch die erste bzw. zweite Aufbewahrungs- und Freisetzungseinheit temporär räumlich voneinander trennbar bzw. getrennt sind, wobei die ersten und zweiten Wandungen unter Einwirkung der vaginalen Temperatur- und/oder Feuchtigkeitsbedingungen zumindest partiell unter Freisetzung von Mucopolysaccharid und Säure aus der ersten bzw. zweiten Aufbewahrungs- und Freisetzungseinheit abbaubar sind.

Dabei kann in einer Ausführungsform der zweiten Ausgestaltung eines erfindungsgemäßen Vaginalapplikators vorgesehen sein, dass die ersten und zweiten Wandungen abschnittsweise über einen gemeinsamen ersten Wandungsabschnitt verfügen, wobei der erste Wandungsabschnitt unter Einwirkung der vaginalen Temperatur- und Feuchtigkeitsbedingungen langsamer abbaubar ist als die verbleibenden Bereiche von erster und zweiter Wandung.

In einer weiteren Ausführungsform kann dieser Vaginalapplikator gemäß der dritten Ausgestaltung ferner ein Behältnis umfassen, enthaltend die erste und die zweite Aufbewahrungs- und Freisetzungseinheit, wobei die Wandung des Behältnisses unter Einwirkung der vaginalen Temperatur- und Feuchtigkeitsbedingungen schneller abbaubar ist als die erste und zweite Wandung von erster bzw. zweiter Aufbewahrungs- und Freisetzungseinheit.

Geeignete erfindungsgemäße Vaginalapplikatoren gemäß der dritten Ausgestaltung können auch in Form von Vaginalsuppositorien vorliegen.

Erfindungsgemäße Vaginalapplikatoren der ersten, zweiten sowie der dritten Ausgestaltung können ferner über mindestens eine dritte Aufbewahrungs- und Freisetzungseinheit verfügen, enthaltend mindestens einen weiteren Inhaltsstoff.

Bevorzugte organische Säuren, die bei den erfindungsgemäßen Vaginalapplikatoren zum Einsatz kommen, umfassen Milchsäure, Weinsäure, Zitronensäure, Bernsteinsäure oder Mischungen dieser Säuren, gegebenenfalls versetzt mit einer basischen Komponente zur Erzeugung eines Puffers, beispielsweise mit einem Lactatpuffer.

Bei den für die erfindungsgemäßen Vaginalapplikatoren zum Einsatz kommenden Mucopolysacchariden handelt es sich im Allgemeinen um lineare Polysaccharide, die aus sich wiederholenden Disaccharid-Einheiten aufgebaut sind. Die Disaccharid-Einheiten umfassen dabei Ester einer Uronsäure, welche 1,3-glucosidisch mit einem Aminosaccharid wie N-Acetylglucosamin verbunden sind. Diese Disaccharid-Einheiten sind miteinander innerhalb der Polymerkette 1,4-glucosidisch verknüpft. Als geeignete Uronsäuren kann z.B. auf Glucuronsäure, Iduronsäure oder Uronsäure zurückgegriffen werden. Mucopolysaccharide im Sinne der vorliegenden Erfindung umfassen ebenfalls Derivate und Salze von Mucopolysacchariden. In Frage kommen z.B. mit einer geeigneten Säure wie Schwefelsäure oder Essigsäure veresterte Mucopolysaccharidderivate. Unter den geeigneten Salzen seien Natrium- und Kaliumsalze von Mucopolysacchariden genannt, wobei die Natrium- und/oder Kaliumsalze der Hyaluronsäure bevorzugt sind.

Unter den Mucopolysacchariden wird für die erfindungsgemäßen Vaginalapplikatoren bevorzugt auf Hyaluronsäure bzw. Derivate und/oder Salze der Hyaluronsäure zurückgegriffen. Auch quervernetzte Hyaluronsäure, insbesondere Hyalane, kommt in Betracht.

In einer weiteren Ausführungsform zeichnen sich die erfindungsgemäßen Vaginalapplikatoren dadurch aus, dass die erste und/oder zweite Aufbewahrungs- und Freisetzungseinheit mindestens einen weiteren Inhaltsstoff, insbesondere Pflegestoff, z.B. Vitamine und/oder Öle, umfassen.

In einer zweckmäßigen Ausgestaltung kann zudem vorgesehen sein, dass die erste, zweite und/oder dritte Aufbewahrungs- und Freisetzungseinheit mindestens eine Peroxidfreisetzende Substanz enthält.

Mit Hilfe der erfindungsgemäßen Vaginalapplikatoren wird die Gefahr des säurebedingten hydrolytischen Abbaus der Hyaluronsäure stark zurückgedrängt bzw. eliminiert, und das, obwohl mit den erfindungsgemäßen Vorrichtungen Mucopolysaccharid und Säure gleichzeitig appliziert werden. Mit der vorliegenden Erfindung wurde somit überraschend gefunden, dass Mucopolysaccharide und Säuren kombiniert als Vaginaltherapeutikum verabreicht werden können, und zwar ohne hierbei in die Gefahr zugeraten, dass die Mucopolysaccharide, z.B. Hyaluronsäure, einem säurebdingten hydrolytischen Abbau unterliegt. Insbesondere ist es auch möglich, auf Formulierungen zurückgreifen zu können, die nicht mehr über eine rein flüssige bzw. ölige Konsistenz, sondern über eine halbfeste bzw. feste Konsistenz verfügen. Die Applizierung eines kombinierten Vaginaltherapeutikums, enthaltend Mucopolysaccharid und Säure, mit den erfindungsgemäßen Vaginalapplikatoren wird als sehr zuverlässig, sicher und angenehm empfunden. Damit können erstmals auf sehr einfache und nicht als störend oder ungenehm empfundene Weise sowohl ein natürlicher Feuchtegrad als auch ein natürliches Säuremilieu in der Scheide eingestellt werden.

Weitere Merkmale und Vorteile der Erfindung ergeben sich aus der nachgehenden Beschreibung, in der bevorzugte Ausführungsformen der Erfindung beispielhaft anhand schematischer Zeichnungen erläutert sind. Hierbei zeigen:
- Figur 1: eine schematische seitliche Schnittansicht eines erfindungsgemäßen Vaginalapplikators gemäß der ersten Ausgestaltung;
- Figur 2: eine weitere Ausführungsform eines erfindungsgemäßen ersten Ausgestaltung;
- Figur 3: die schematische seitliche Schnittansicht eines erfindungsgemäßen Vaginalapplikators gemäß der zweiten Ausgestaltung; und
- Figur 4: eine schematische seitliche Schnittansicht eines erfindungsgemäßen Vaginalapplikator gemäß der dritten Ausgestaltung.

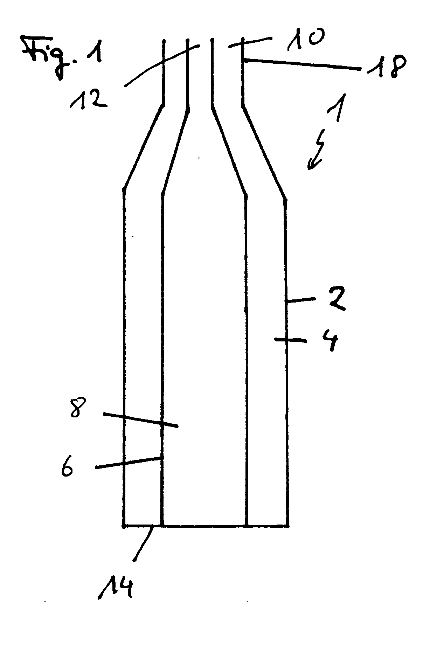

Figur 1 zeigt einen erfindungsgemäßen Vaginalapplikator gemäß der ersten Ausgestaltung 1, umfassend eine erste Aufbewahrungseinheit 2 enthaltend Mucopolysaccharid 4, z.B. Hyaluronsäure, sowie eine zweite Aufbewahrungseinheit 6 enthaltend Säure 8, vorzugsweise Milchsäure. Die erste Aufbewahrungseinheit 2 ist mit einer ersten Auslassöffnung 10 ausgestattet und die zweite Aufbewahrungseinheit 6 mit einer zweiten Auslassöffnung 12. Die zweite Auslassöffnung 12 befindet sich wie das zweite Aufbewahrungsbehältnis 6 innerhalb der ersten Auslassöffnung bzw. der ersten Aufbewahrungseinheit 2. Die Wandungen von erster und zweiter Aufbewahrungseinheit 2 und 6 sind in der dargestellten Ausführungsform aus flexiblem Material bzw. einer dickwandigen Polymerfolie gebildet. Aus Praktikabilitätsgründen kann es vorteilhaft sein, den Boden 14 des Vaginalapplikators 1 fester auszugestalten, damit dieser als Standfläche fungieren kann.

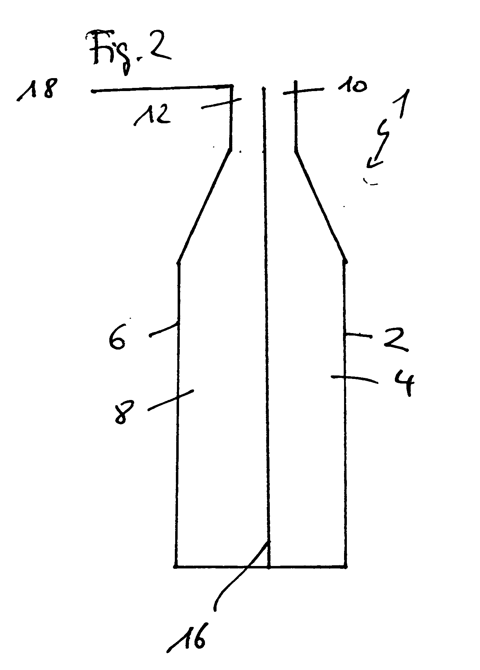

Figur 2 zeigt eine weitere Ausführungsform eines erfindungsgemäßen Vaginalapplikators 1 gemäß der ersten Ausgestaltung. Bei dieser Ausführungsform liegt anders als bei der Ausführungsform gemäß Figur 1 die zweite Aufbewahrungseinheit 6 nicht innerhalb der ersten Aufbewahrungseinheit 2 vor, sondern erste und zweite Aufbewahrungseinheit sind nebeneinander angeordnet und verfügen über eine gemeinsame Trennwand 16. Demgemäß liegen auch die erste und zweite Auslassöffnung 10, 12 nebeneinander vor. Sowohl bei der Ausführungsform gemäß Figur 1 als auch bei der Ausführungsform gemäß Figur 2 eines Vaginalapplikators 1 gemäß einer ersten Ausgestaltung sind die erste und die zweite Auslassöffnung 10, 12 derart ausgebildet, dass sie jeweils eine Ausgabeeinheit 18 bilden, welche ausgerichtet und geeignet ist, um Mucopolysaccharid und Säure gemeinsam in die Vagina zu applizieren.

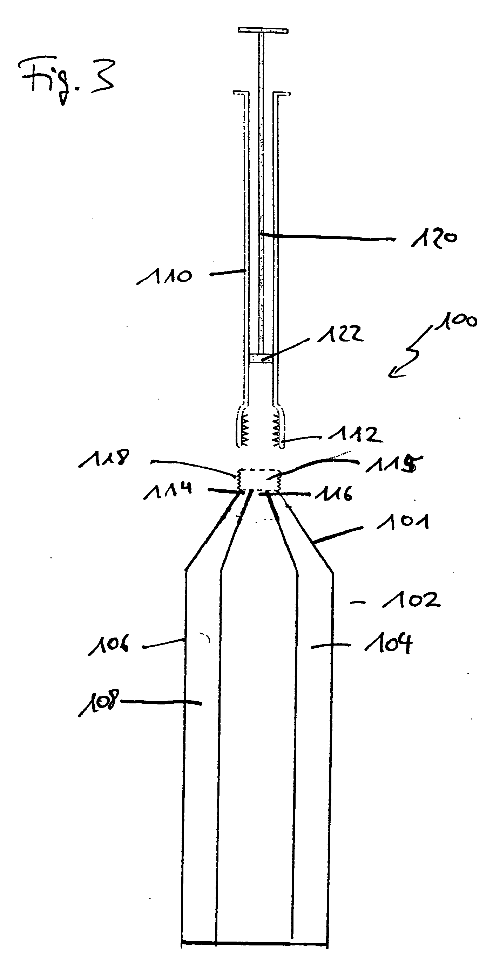

Figur 3 zeigt einen erfindungsgemäßen Vaginalapplikator 100 gemäß der zweiten Ausgestaltung. Diese liegt in Form eines Applikationskits vor, umfassend ein kombiniertes Aufbewahrungsbehältnis 101, welches eine erste Aufbewahrungseinheit 102, enthaltend Mucopolysaccharid 104, beispielsweise Hyaluronsäure, und eine zweite Aufbewahrungseinheit 106, enthaltend Säure, insbesondere Milchsäure, sowie eine zweite Applikationshilfe 110 aufweist.

Die zweite Applikationshilfe 110 liegt separat bzw. separierbar von dem kombinierten Aufbewahrungsbehältnis 101 vor und kann z.B., wie in Figur 3 dargestellt, über einen Schraubverschluss mit dem Aufbarungsbehältnis 101 verbunden werden. In der dargestellten Ausführungsform wird die zweite Applikationshilfe 110 im Bereich ihres Öffnungsaufsatzes 112 über ein Schraubgewinde 115 mit der Außenseite der ersten Auslassöffnung 114 verbunden. Auch bei dem Vaginalapplikator 1 gemäß der zweiten Ausgestaltung liegt, ähnlich wie bei der Ausführungsform gemäß Figur 1, die zweite Auslassöffnung 116 der zweiten Aufbewahrungseinheit 106 innerhalb der ersten Auslassöffnung 114 vor. Die erste und die zweite Auslassöffnung 114, 116 bilden eine Ausgabeeinheit 118, welche nicht derart ausgeführt ist, dass eine einwandfreie und zuverlässige gemeinsame Applizierung von Mucopolysaccharid und Säure ohne weiteres gelingt. Hierfür ist die zweite Applikationshilfe 110 vorgesehen. Diese zweite Applikationshilfe 110 ist hierbei mit einer Applikationsvorrichtung 120 in Form eines in der zylinderförmigen Applikationshilfe vorliegenden Stempels ausgeführt, der innerhalb der zweiten Applikationshilfe 110 frei bewegbar ist. Der Stempelkopf 122 liegt dicht an der Innenwandung der zweiten Applikationshilfe 110 an. Indem man Mucopolysaccharid und Säure aus dem Aufbewahrungsbehältnis 101 herausdrückt, wird eine Mischung aus Mucopolysaccharid und Säure bei auf die Ausgabeeinheit aufgesetzter Applikationshilfe in diese Applikationshilfe überführt. Liegt ausreichend Material in der Applikationshilfe vor, kann diese ohne weiteres von dem Aufbewahrungsbehältnis 101 getrennt werden. Mit Hilfe dieser Applikationshilfe 110 kann sodann die Mischung aus Mucopolysaccharid und Säure über den Öffnungsaufsatz 112 in die Vagina appliziert werden.

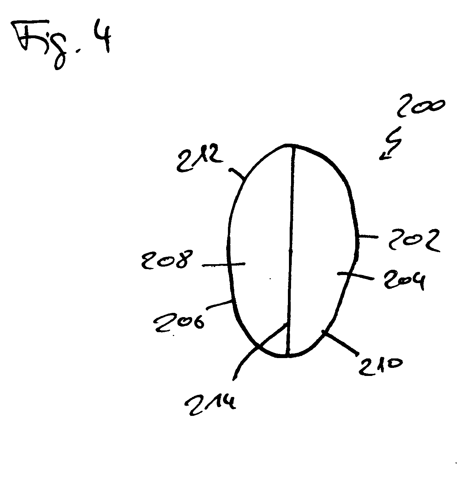

Figur 4 entnimmt man einen Vaginalapplikator 200 gemäß der dritten Ausgestaltung. Dieser Vaginalapplikator verfügt über eine erste Aufbewahrungs- und Freisetzungseinheit 202 enthaltend ein Mucopolysaccharid, beispielsweise Hyaluronsäure, und eine zweite Aufbewahrungs- und Freisetzungseinheit 206 enthaltend eine Säure, vorzugsweise Milchsäure, 208. Die ersten und zweiten Aufbewahrungs- und Freisetzungseinheiten 202 und 206 sind durch erste und zweite Wandungen 210 und 212 begrenzt. Diese ersten und zweiten Aufbewahrungs- und Freisetzungseinheiten 202 und 208 verfügen zudem über einen gemeinsamen ersten Wandungsabschnitt 214. Die ersten und zweiten Wandungen 210 und 212 sind unter den vaginalen Temperatur- und/oder Feuchtigkeitsbedingungen zumindest bereichsweise unter Freisetzung von Mucopolysaccharid und Säure abbaubar. Demnach wird der Vaginalapplikator gemäß der dritten Ausgestaltung, beispielsweise als Vaginalsuppositorium, in die Vagina eingeführt. Dort kommt es zur Zersetzung der ersten und zweiten Wandung unter gleichzeitiger Freigabe von Mucopolysaccharid und Säure. Hierbei ist der erste Wandungsabschnitt 214 derart ausgestaltet, dass dieser unter den vaginalen Temperaturen- und/oder Feuchtigkeitsbedingungen langsamer abgebaut wird als die verbleibenden Bereiche von erster und zweiter Wandung 210, 212.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Vaginalapplikator, umfassend
mindestens eine erste Aufbewahrungseinheit, enthaltend mindestens ein Mucopolysaccharid, insbesondere Hyaluronsäure und/oder ein Derivat und/oder Salz der Hyaluronsäure, mit einer ersten Auslassöffnung und mindestens eine zweite Aufbewahrungseinheit, enthaltend mindestens eine Säure, insbesondere organische Säure, mit einer zweiten Auslassöffnung, wobei die erste und die zweite Auslassöffnung benachbart angeordnet sind unter Ausbildung einer ersten Ausgabeeinheit zur gemeinsamen Applikation von Mucopolysaccharid und Säure in die Vagina.

2. Vaginalapplikator nach Anspruch 1, **dadurch gekennzeichnet, dass**
die erste und zweite Auslassöffnung nebeneinander vorliegen, insbesondere eine gemeinsame Trennwand aufweisen.

3. Vaginalapplikator nach Anspruch 1 oder 2, **dadurch gekennzeichnet, dass**
die erste Aufbewahrungseinheit zumindest bereichsweise innerhalb der zweiten Aufbewahrungseinheit angeordnet ist und/oder dass die erste Auslassöffnung zumindest bereichsweise im Umfang der zweiten Auslassöffnung vorliegt oder dass die zweite Aufbewahrungseinheit zumindest bereichsweise innerhalb der ersten Aufbewahrungseinheit vorliegt und/oder dass die zweite Auslassöffnung zumindest bereichsweise im Umfang der ersten Auslassöffnung vorliegt.

4. Vaginalapplikator nach einem der vorangehenden Ansprüche, ferner umfassend eine erste Applikationshilfe, die mit der ersten Ausgabeeinheit verbunden oder verbindbar ist.

5. Vaginalapplikator nach Anspruch 4, **dadurch gekennzeichnet, dass**
die erste Ausgabeeinheit und/oder die erste Applikationshilfe mindestens ein Mischelement enthält.

6. Vaginalapplikator, umfassend ein kombiniertes Aufbewahrungsbehältnis, enthaltend eine erste Aufbewahrungseinheit, enthaltend mindestens ein Mucopolysaccharid, insbesondere Hyaluronsäure und/oder ein Derivat und/oder Salz der Hyaluronsäure, mit einer ersten Auslassöffnung und mindestens eine zweite Aufbewahrungseinheit, enthaltend mindestens eine Säure, insbesondere organische Säure, mit einer zweiten Auslassöffnung, wobei diese erste und zweite Auslassöffnungen benachbart angeordnet sind unter Ausbildung einer zweiten Ausgabeeinheit,
sowie mindestens eine zweite Applikationshilfe, umfassend einen Öffnungsaufsatz, der reversibel dichtend mit der zweiten Ausgabeeinheit verbunden oder verbindbar ist, und über mindestens eine Applikatorvorrichtung verfügt zur gemeinsamen Applikation von Mucopolysaccharid und Säure in die Vagina über den Öffnungsaufsatz.

7. Vaginalapplikator, enthaltend
mindestens eine erste Aufbewahrungs- und Freisetzungseinheit, enthaltend mindestens ein Mucopolysaccharid, insbesondere Hyaluronsäure und/oder ein Derivat und/oder Salz der Hyaluronsäure, mit einer ersten Wandung und mindestens eine zweite Aufbewahrungs- und Freisetzungseinheit, enthaltend mindestens eine Säure, insbesondere organische Säure, mit einer zweiten Wandung, wobei Mucopolysaccharid und Säure durch die erste bzw. zweite Aufbewahrungs- und Freisetzungseinheit temporär räumlich voneinander trennbar bzw. getrennt sind, wobei die ersten und zweiten Wandungen unter Einwirkung der vaginalen Temperatur- und/oder Feuchtigkeitsbedingungen zumindest partiell unter Freisetzung von Mucopolysaccharid und Säure aus der ersten bzw. zweiten Aufbewahrungs- und Freisetzungseinheit abbaubar sind.

8. Vaginalapplikator nach Anspruch 7, **dadurch gekennzeichnet, dass**
die ersten und zweiten Wandungen abschnittsweise über einen gemeinsamen ersten Wandungsabschnitt verfügen, wobei der erste Wandungsabschnitt unter Einwirkung der vaginalen Temperatur- und Feuchtigkeitsbedingungen langsamer abbaubar ist als die verbleibenden Bereiche von erster und zweiter Wandung.

9. Vaginalapplikator nach Anspruch 7 oder 8, ferner umfassend
ein Behältnis enthaltend die erste und die zweite Aufbewahrungs- und Freisetzungseinheit, wobei die Wandung des Behältnisses unter Einwirkung der vaginalen Temperatur- und Feuchtigkeitsbedingungen schneller abbaubar ist als die erste und zweite Wandung von erster bzw. zweiter Aufbewahrungs- und Freisetzungseinheit.

10. Vaginalapplikator nach einem der vorangehenden Ansprüche, **gekennzeichnet durch** mindestens eine dritte Aufbewahrungs- und Freisetzungseinheit, enthaltend mindestens einen weiteren Inhaltsstoff.

11. Vaginalapplikator nach einem der Ansprüche 7 bis 10, **dadurch gekennzeichnet, dass** dieser ein Vaginalsuppositorium darstellt.

12. Vaginalapplikator nach einem der vorangehenden Ansprüche, **dadurch gekennzeichnet, dass**
die organische Säure ausgewählt ist aus der Gruppe, bestehend aus Milchsäure, Weinsäure, Zitronensäure, Bernsteinsäure oder einer Mischung dieser Säuren, gegebenenfalls versetzt mit einem Puffer.

13. Vaginalapplikator nach einem der vorangehenden Ansprüche, **dadurch gekennzeichnet, dass**
das Salz der Hyaluronsäure das Natrium- und/oder das Kaliumsalz oder dass die Hyaluronsäure quervernetzte Hyaluronsäure, insbesondere Hyalane, umfasst.
